# EUROPÄISCHE PATENTANMELDUNG

(11) **EP 0 811 351 A1**
(43) Veröffentlichungstag der Anmeldung: **10.12.1997**
(21) Anmeldenummer: 97108680.6
(22) Anmeldetag: 30.05.1997
(51) Int. Cl.: A61B 3/06

(54) **Vorrichtung zur Bestimmung der Reihenfolge von Farbfleckscheiben eines Farbflecktests**

(30) Priorität: 05.06.1996 AT 817/96; 05.06.1996 AT 466/97
(71) Anmelder: Daxer, Albert, Dipl.-Ing. Dr., 1120 Wien (AT)
(72) Erfinder: Daxer, Albert, Dipl.-Ing. Dr., 1120 Wien (AT)

(57) **Zusammenfassung**

Zweck der Erfindung ist die automatisierte Bestimmung der Reihenfolge von Farbknöpfen (2) in einem Farbfleck- bzw. Farbanordnungstest (z.B. Farnsworth-Munsell 100-hue Test) zur Bestimmung der Farbtüchtigkeit von Menschen. An oder in den Farbknöpfen (2) werden dazu Stellen (4) festgelegt, an welchen Magnete (5) angebracht werden können. Die für jeden Farbknopf (2) verschiedene Verteilung von Magneten (5) und Leerstellen (Nichtmagneten) (15) über die festgelegten Stellen (4) in den Farbknöpfen (2) codiert die Farbknöpfe (2). Eine andere Art der Codierung kann durch die Erzeugung unterschiedlich starker Magnetfelder durch die Magnete (5) im Farbknopf (2) erreicht werden. In der Auflagefläche für die Farbknöpfe (2) im Legekasten (1) sind an geeigneten Stellen Magnetfelddetektoren (6) angebracht. Mittels dieser Magnetfelddetektoren (6) und einer entsprechenden Elektronik wird die Reihenfolge der Farbknöpfe (2) bestimmt. Durch eine symmetriebrechende Struktur (7) an den Farbknöpfen (2) oder durch geeignete Magnete (5) unterschiedlicher Stärke an oder in den Farbknöpfen (2) wird eine, gegenüber Verdrehung von Farbknöpfen (2), sichere Auswertung ermöglicht.

## Beschreibung

### Zweck der Erfindung:

Die Erfindung betrifft eine Vorrichtung zur Untersuchung der Farbtüchtigkeit des visuellen Systems beim Menschen. Im besonderen handelt es sich dabei um eine Vorrichtung zur automatisierten Ermittlung der Reihenfolgen von Farbknöpfen bei Farbfleck- bzw. Farbanordnungstests wie z.B. dem Farnsworth-Munsell 100-hue Test.

### Grundlagen:

Zur Ermittlung der Farbtüchtigkeit des Menschen gibt es verschiedene Verfahren. Eines stellt die Durchführung von Farbfleck- bzw. Farbanordnungstests dar, welches in der Zeitschrift: Z. prakt. Augenheilkunde, 10 (1989), Seiten 29 bis 35 beschrieben ist. Das Prinzip dieses Tests besteht darin, dem Probanden eine Reihe verschiedener Farbflecke anzubieten und ihn aufzufordern, diese, nach allmählicher Farbänderung abgestuft, in der richtigen Reihenfolge anzuordnen. Bei farbsinngestörten Menschen treten dabei für die entsprechende Störung mehr oder weniger charakteristische Verwechslungen auf.

Der bekannteste Vertreter dieser Art von Farbsinntests ist der Farnsworth-Munsell 100-hue Test, welcher aus 85 beweglichen und 8 fixierten farblichen Scheibchen mit einem Durchmesser von je 5mm besteht. Insgesamt repräsentieren diese 93 Scheiben einen Ausschnitt aus dem natürlichen Farbspektrum sowie aus dem Bereich der Purpurfarben. Die Scheiben werden auf Legekästen verteilt. Aneinandergelegt nach dem Kriterium, möglichst ähnliche Farben nacheinander anzuordnen, ergibt sich ein geschlossener Farbkreis. Jeder Scheibe, und damit jedem Farbfleck ist eine Zahl zugeordnet, welche die Berechnung von Teilfehlerzahlen und einer Gesamtfehlerzahl erlaubt. Die Gesamtfehlerzahl ist insbesondere bei erworbenen Farbsinnstörungen ein sehr guter Parameter für die Verlaufskontrolle.

Die Ermittlung der Fehlerzahlen ist relativ zeitaufwendig und beinhaltet selbst eine gewisse Fehlerwahrscheinlichkeit. Es wurde versucht, den Aufwand mittels geeigneter Computerprogramme zu verringern, wobei jedoch die vom Probanden gelegte Reihenfolge erst nach deren manueller Eingabe in den Computer zur Berechnung der Fehlerzahlen herangezogen werden kann. Dies bewirkt zwar eine gewisse Zeitersparnis bei der Berechnung - die Eingabe selbst ist jedoch auch zeitaufwendig und bedarf vor allem einer hohen Konzentration um Eingabefehler gering zu halten.

### Stand der Technik:

Als Stand der Technik auf dem Gebiet der Erfindung gelten die Verfahren und Vorrichtungen von Donaldson und Hill. Donaldson entwickelte eine Vorrichtung zur automatisierten Ermittlung der Farbreihenfolge der Farbfleckscheiben, welche in der Zeitschrift: J. Optical Society of America, Vol. 67, No. 2, February 1977, Seiten 248 bis 249 beschrieben ist. Dabei wurden in den Knöpfen, welche die Farbscheiben enthalten, je ein Widerstand und eine Zenerdiode angebracht und parallel geschalten. Über geeignete Kombinationen dieser Schaltelemente konnten die Farbscheiben geeignet codiert werden und , durch elektrische Steckverbindungen (Klinkenstecker), welche an der Unterseite der Farbknöpfe angebracht waren, konnte die gelegte Reihenfolge der Farbscheiben ermittelt werden. Dieses Verfahren besitzt folgende Nachteile:
1) Bei der Auswahl der Farbknöpfe durch den Probanden (Patienten) liegen die Knöpfe üblicherweise in ungeordneter Weise, mit dem Farbfleck an der Oberseite, vor dem Probanden (Patienten) auf einer Tischfläche. Beim normalen Farnsworth-Munsell 100-hue Test ist dies kein Problem, da die Unterseite der Knöpfe eben ist. Bei der von Donaldson vorgestellten automatisierten Version, ist ein einfaches Auflegen der noch ungeordneten Farbknöpfe auf der Tischplatte nicht möglich, da die Farbknöpfe keine ebene Unterfläche mehr besitzen und daher die einzelnen Farbknöpfe nicht mehr stabil, mit der Farbfläche nach oben vor dem Probanden (Patienten) auf den Tisch gelegt werden können. Vielmehr liegen die Farbknöpfe, wegen des an der Unterseite angebrachten Steckers, instabil, quasi wie ein Kreise auf der ebenen Tischfläche und die Farbflächen sind nicht nach oben sondern in eine beliebige Richtung orientiert. Dies führt für den Probanden (Patienten) zu einer erheblichen Behinderung bei der Farbauswahl.
2) Für den bequemen Ablauf des Anordnungsvorganges im Legekasten sollten die Farbfleckscheiben in der Anordnungsrichtung verschieblich sein, um, wenn der Patient zwischen zwei oder mehreren bereits gelegten (angeordneten) Farbknöpfen noch einen Farbknopf anordnen will, einen entsprechenden Platz dafür, durch Verschiebung der bereits gelegten (angeordneten) Farbknöpfe, schaffen zu können. Im Falle der von Donaldson vorgelegten Entwicklung ist dies nicht möglich. Es müßten dabei alle bereits angeordneten Farbknöpfe aus den Steckverbindungen entnommen werden, und in die, der notwendigen Versetzung entsprechenden Positionen gesteckt werden. Dann erst kann der Proband (Patient) den gewünschten Farbknopf in die freigewordene Position stecken. Kommt der Patient, nachdem er die so angeordnete Teilfarbfolge betrachtet hat zu dem Schluß, daß der nunmehr dazwischen abgebrachte Farbknopf in seiner Farbe nun doch nicht an diese Stelle paßt, was häufig der Fall ist, muß er alle durchgeführten Umsteckmanöver wieder rückgängig machen.
3) Die Steckverbindung ist nicht nur ein elektrisches Teil, sondern auch ein mechanisches Teil, und somit verschleißanfällig.
4) Die Farbknöpfe enthalten elektrische Bauteile, welche über eine relativ begrenzte Lebensdauer verfügen. Sollte beispielsweise die Zenerdiode im Inneren des Farbknopfes kaputtgehen, müßte diese ersetzt werden, was mit einem nicht zu vernachlässigbaren Aufwand verbunden wäre.

Um einige der beschriebenen Nachteile möglichst zu umgehen, hat Hill eine Vorrichtung zur automatisierten Bestimmung der gelegten Farbreihenfolge bei Farbanordnungtests entwickelt, welches über eine Art Barcodelesesystem funktioniert. Diese Vorrichtung ist in der WO 88/03776 beschrieben. An den Farbknöpfen wurde dabei jeweils ein Barcode, vorzugsweise bestehend aus konzentrischen Ringen, angebracht, um, mit einem Lesegerät, jeden Farbknopf, unabhängig von seiner Orientierung (Verdrehung um die senkrechte Achse), identifizieren zu können. Nachteile dieses Apparates sind:
1) Ein entsprechendes Lesegerät muß über die Farbknöpfe scannen, was einer beweglichen, mechanischen Vorrichtung bedarf. Dadurch ergibt sich eine gewisse Störungsanfälligkeit bzw. ein entsprechender Wartungsbedarf. Dies um so mehr, je mehr Untersuchungen pro Zeiteinheit durchgeführt werden.
2) Die Mechanik bedarf hoher Qualität und ist daher teuer.
3) Barcodesysteme sind fehleranfällig (nicht jeder Leseversuch gelingt).
4) Die Lesevorrichtung kann verschmutzen, was zu erhöhter Wartungsnotwendigkeit führt.

Der Erfindung liegt die Aufgabe zugrunde, eine Vorrichtung zur einfachen Bestimmung der Reihenfolge von Farbknöpfen bei einem Farbfleck- bzw. Farbanordnungstest wie z.B. dem Farnsworth-Munsell 100-hue Test anzugeben, die einen einfachen Aufbau aufweist und einfach handhabbar ist.

Diese Aufgabe wird erfindungsgemäß durch die Merkmale des Patentanspruches 1 gelöst.

Vorteilhafte Ausbildungen der Erfindung sind in den abhängigen Patentansprüchen angegeben.

### Beschreibung der Erfindung:

Ein Ausführungsbeispiel der Erfindung wird anhand der Zeichnungen näher erläutert.

Es zeigen:
- Figur 1: eine erfindungsgemäße Vorrichtung in der Gesamtansicht,
- Figur 2: einen vergrößerten Ausschnitt der Vorrichtung aus Figur 1 und
- Figur 3: die Ansicht der Unterseite von zwei Farbknöpfen.

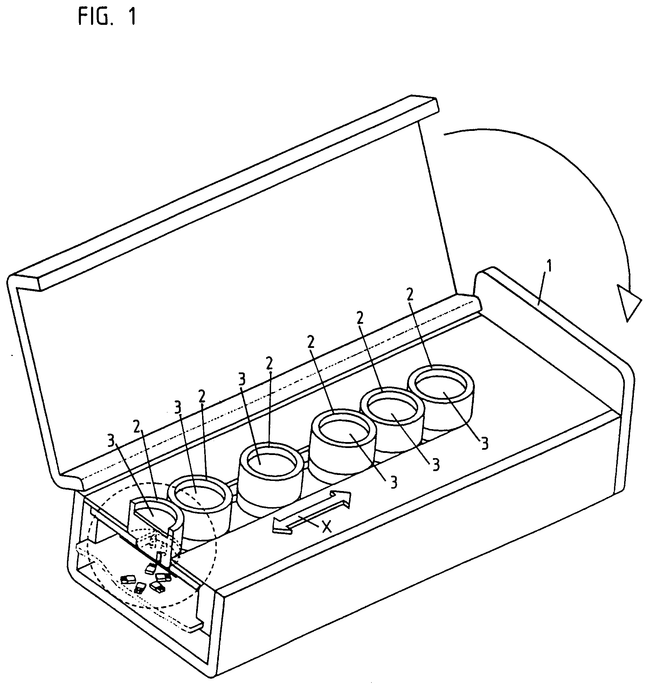

Die in Figur 1 dargestellte Vorrichtung besteht im wesentlichen aus einem Legekasten 1, in welchen Farbknöpfe 2 eingelegt bzw. aufgelegt werden können. Vorzugsweise an der Oberseite der Farbknöpfe 2 sind Farbflecke 3 angebracht. An oder in den Farbknöpfen 2 sind definierte Stellen 4 (Orte) festgelegt, an welchen Magnete 5 angebracht werden können. An zu den definierten Stellen 4, im Legekasten 1 korrespondierenden Stellen sind Magnetfelddetektoren 6 (z.B. Hall-Detektoren) angebracht. Die Farbknöpfe 2 besitzen eine symmetriebrechende geometrische Veränderung, vorzugsweise eine Stufe 7, welche dafür sorgt, daß die Farbknöpfe 2 beim Einlegen in den Legekasten 1 so ausgerichtet werden, daß die potentiellen Orte 4 der Magnete 5 im Farbknopf 2 über den korrespondierenden Magnetfelddetektorpositionen im Legekasten 1 zu liegen kommen. Die für jeden Farbknopf 2 unterschiedliche Verteilung von Magneten 5 und Leerstellen 15 (Nichtmagneten) über die definierten Stellen 4 codiert in eindeutiger Weise den entsprechenden Farbknopf 2.

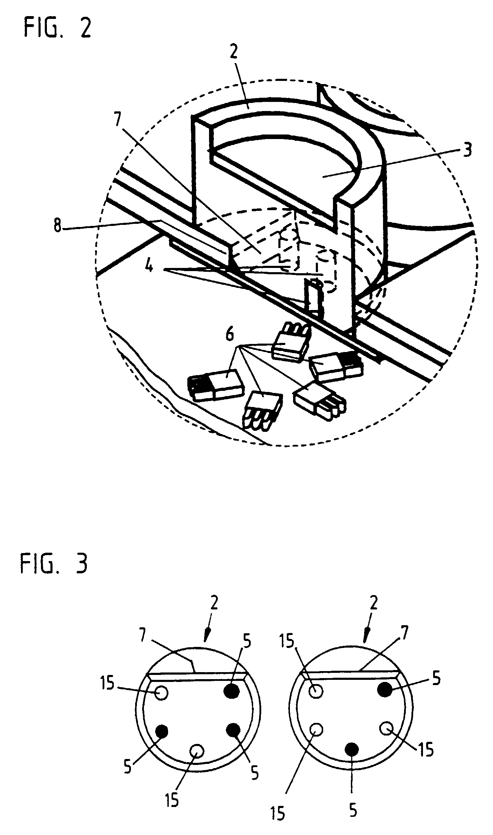

In der vergrößerten Darstellung gemäß Figur 2 ist ersichtlich, daß die definierten Stellen 4 der Farbknöpfe 2 besonders vorteilhaft in Form von Bohrungen vorgesehen sind, in welche stabförmige Magnete 5 auf einfache Weise eingebracht werden können. In Figur 2 ist ebenfalls die Stufe 7 der Farbknöpfe 2 gut erkennbar. Die Stufe 7 besitzt eine Kante, welche in der gewünschten Verschieberichtung X der Farbknöpfe 2 verläuft. Im Legekasten 1 ist ein in Verschieberichtung X verlaufender Absatz 8 vorgesehen, mit dem die Stufe 7 beim Einlegen der Farbknöpfe 2 formschlüssig zusammenwirkt.

Die Codierungsbeispiele in Figur 3 zeigen die geöffnete Unterseite zweier verschiedener Farbknöpfe 2, wobei die schwarzen Kreisinhalte Orte 4 mit einem Magneten 5 und die weißen Kreisinhalte Orte 4 einer Leerposition 15 ohne Magnet 5 symbolisieren. Die, an zu den definierten Stellen im Legekasten 1 korrespondierenden Stellen angebrachten, Magnetfelddetektoren 6 stellen fest, an welcher der definierten Stellen 4 im Farbknopf 2 ein Magnet 5 vorhanden ist und an welchen Stellen 4 nicht. Diese Information wird dann über eine geeignete, mit den Magnetfelddetektoren verbundene Elektronik (Auswerteeinheit) verarbeitet und vorzugsweise, zur weiteren Auswertung, an ein Computersystem übertragen. Die Magnetcodierung der Farbknöpfe 2 wird durch die Magnetfelddetektoren 6 erfaßt und dadurch jeder der aufgelegten Farbknöpfe 2 identifiziert. Aufgrund dieser Identifikation bestimmt das Computersystem die Reihenfolge der aufgelegten Farbknöpfe 2. Um die definierten Stellen 4 für die Anbringung der Magnete 5 in den Farbknöpfen 2 über den korrespondierenden Stellen für die Magnetfelddetektoren 6 im Legekasten 1 positionieren zu können, besitzen die Farbknöpfe 2 eine symmetriebrechende Veränderung 7, welche die Ausrichtung der Farbknöpfe 2 durch die Möglichkeit des Ineinanderfügens dieser Elemente 7, 8 fixiert.

Eine andere Möglichkeit die Ermittlung der Reihenfolge der Farbknöpfe sicher gegen Verdrehung der Farbknöpfe zu machen, wäre die Einbringung eines oder mehrerer Magnete mit unterschiedlicher Magnetstärke in den Farbknopf und entsprechend korrespondierender Magnetfelddetektoren in den Legekasten.

Die Erfindung hat gegenüber den bisher bekannten Vorrichtungen folgende Vorteile:
1) Zur automatisierten Bestimmung der Reihenfolgen der Farbknöpfe 2 sind keine mechanisch beweglichen Teile und Vorrichtungen notwendig.
2) Die Informationsübertragung zwischen den Farbknöpfen 2 und der Elektronik 6 im Legekasten 1 findet ohne elektrische oder mechanische Kontakte, also kontaktlos statt.
3) Die Farbknöpfe 2 sind (solange nicht alle Farbknöpfe 2 angeordnet sind) im Legekasten 1 verschieblich.
4) Die Farbknöpfe 2 sind trotz der symmetriebrechenden Veränderung (vorzugsweise Stufe 7 an Unterseite) stabil auf eine ebene Unterlage zu positionieren.
5) Der Aufbau und die entsprechenden Bauteile sind vergleichsweise kostengünstig.
6) Die Farbknöpfe 2 enthalten keine verschleißbaren elektronischen oder mechanischen oder optischen Bauteile.
7) Keine Wartungsprobleme.
8) Extrem niedriger Stromverbrauch.
9) Einfache Handhabung für Patient und Untersucher.
10) Keine Stromversorgung für die Farbknöpfe notwendig.

Eine zweite, nicht dargestellte Ausführungsform ist dadurch gekennzeichnet, daß vorzugsweise im geometrischen Zentrum der Farbknöpfe ein Magnet oder Magnetsystem angebracht ist, welches den individuellen Farbknopf durch die Stärke des dadurch erzeugten Magnetfeldes an der korrespondierenden Magnetfelddetektorposition im Legekasten codiert. Die Informationen über die, an einer bestimmten Farbknopfposition im Legekasten herrschenden Magnetfeldstärke wird durch eine nachfolgende Auswerteeinheit ausgewertet und dargestellt. Diese Ausführungsform erlaubt eine verdrehungssichere Identifikation der Farbknöpfe ohne symmetriebrechende Veränderungen (Stufen, Abschrägungen, etc.) an der Geometrie der Farbknöpfe. Bei dieser Ausführungsform wird die Information über den jeweiligen Farbknopf nicht durch die Anordnung von Magneten im Farbknopf, sondern durch die Stärke des, von einem oder mehreren Magneten im Farbknopf erzeugten Magnetfeldes codiert.

In nicht gezeigter Weise ist auch eine Kombination der beiden beschriebenen Ausführungsformen möglich, bei der also die Farbknöpfe durch die Anordnung - also den Ort - von Magneten und die Stärke der Magnete codiert sind. Zur Codierung kann auch die Richtung des Magnetfeldes der Magnete herangezogen werden.

## Patentansprüche

1. Vorrichtung zur Bestimmung der Reihenfolge von Farbknöpfen (2) eines Farbflecktestes zur Bestimmung der Farbtüchtigkeit von Menschen, dadurch gekennzeichnet, daß an oder in zumindest einem Farbknopf (2) zumindest ein Magnet (5) angebracht ist.

2. Vorrichtung nach Anspruch 1, dadurch gekennzeichnet, daß an oder in den Farbknöpfen (2) ein oder mehrere Stellen (4) festgelegt sind, an denen je zumindest ein Magnet (5) angebracht werden kann.

3. Vorrichtung nach Anspruch 1 oder 2, dadurch gekennzeichnet, daß vorzugsweise an, zu möglichen Magnetpositionen im Farbknopf (2) korrespondierenden Stellen im Bereich der Auflageflächen der Farbknöpfe (2) in einem Legekasten (1), Magnetfelddetektoren (6) angebracht sind.

4. Vorrichtung nach einem der Ansprüche 1, 2 oder 3, dadurch gekennzeichnet, daß die Codierung der Farbknöpfe (2) durch die, bei jedem Farbknopf (2) unterschiedliche Zuteilung von Magneten (5) einerseits und Leerstellen (Nichtmagnete) (10) andererseits auf die für die mögliche Anbringung von Magneten (5) festgelegten Stellen (4) an oder in den Farbknöpfen (2) geschieht.

5. Vorrichtung nach einem der Ansprüche 1, 2, 3 oder 4, dadurch gekennzeichnet, daß die Codierung der Farbknöpfe durch Anbringung von Magneten dergestalt geschieht, daß die Stärke des Magnetfeldes von je zwei verschiedenen Farbknöpfen, an den zu den jeweiligen Farbknopfpositionen gehörigen Magnetfelddetektorpositionen, unterschiedlich ist.

6. Vorrichtung nach einem der Ansprüche 1, 2, 3, 4 oder 5, dadurch gekennzeichnet, daß die Farbknöpfe(2) eine symmetriebrechende Struktur, welche vorzugsweise in Form einer Stufe (7) oder Abschrägung ausgeführt ist, aufweist.

7. Vorrichtung nach einem der Ansprüche 1, 2, 3, 4, 5 oder 6, dadurch gekennzeichnet, daß der Ablagebereich für die Farbknöpfe (2) im Legekasten (1), eine zur symmetriebrechenden Struktur (7) der Farbknöpfe (2) komplementäre Struktur (8) aufweist.
